# EUROPEAN PATENT APPLICATION

(11) **EP 3 020 479 A1**
(43) Date of publication of application: **18.05.2016**
(21) Application number: 15193744.8
(22) Date of filing: 09.11.2015
(51) Int. Cl.: B01J 35/00, C03C 17/245, G02F 1/1333

(54) **DISPLAY APPARATUS COMPRISING A PHOTOCATALYTIC LAYER AND METHOD OF MANUFACTURING THE SAME**

(30) Priority: 13.11.2014 KR 20140157792
(71) Applicant: Samsung Electronics Co., Ltd., Gyeonggi-do 16677 (KR)
(72) Inventor: SHIN, Seong Hwan, Seoul (KR); WON, Ji Na, Gyeonggi-do (KR); YOO, Hyong Jun, Gyeonggi-do (KR); LEE, Gun Hyo, Gyeonggi-do (KR); LEE, Seo Joon, Gyeonggi-do (KR); JUNG, Min Chul, Gyeonggi-do (KR); CHO, Jin Hyun, Seoul (KR)
(74) Representative: Walaski, Jan Filip

(57) **Abstract**

A display apparatus (300) in which a photocatalyst thin film (370) is formed is provided. The display apparatus (300) includes a housing (305) forming an exterior of the display apparatus (300), a display panel (320) coupled to the housing (305), and a photocatalyst thin film (370) which is formed on a surface of one of the housing (305) and the display panel (320).

## Description

The present invention relates to a display apparatus having a photocatalyst function and a method of manufacturing the same.

A display apparatus, which is an apparatus including a display unit on which images are displayed, can include a television or a monitor.

A display apparatus can include components such as a display panel on which images are displayed, a backlight unit which emits light to the display panel, and the like.

A screen and an exterior of a display apparatus, specifically, a display apparatus to be used in public places, can easily be contaminated by organic materials, fine dust, or the like. Thus, in order to maintain the cleanliness of the display apparatus, such various organic materials, fine dust, etc., should be cleaned from the display as soon as possible.

One or more exemplary embodiments may provide a display apparatus including a photocatalyst thin film applied to a display panel or an exterior part of the display apparatus.

One or more exemplary embodiments may provide a method of manufacturing a display apparatus including a photocatalyst thin film is formed on a display panel or an exterior part of the display apparatus.

Additional exemplary aspects and advantages will be set forth in part in the description which follows and, in part, will be obvious from the description, or may be learned by practice of the exemplary embodiments described herein.

In accordance with one aspect of an exemplary embodiment, a display apparatus includes a housing which forms an exterior of the display apparatus, a display panel coupled to the housing, and a photocatalyst thin film formed on a surface of at least one of the housing and the display panel.

The photocatalyst thin film may include at least one of a titanium oxide (TiO2) thin film and a nitrogen-doped titanium oxide (TiO2-xNx) thin film.

The display apparatus may include at least one display apparatus selected from a group of a liquid crystal display (LCD) apparatus, an organic light-emitting display (OLED) apparatus, and a quantum dot display apparatus.

The housing may be formed of a metal component or a plastic component.

The metal component may include an aluminum alloy component.

In accordance with an aspect of another exemplary embodiment, a display apparatus includes a housing which forms an exterior of the display apparatus, a display panel coupled to the housing, and a photocatalyst thin film formed on a surface of the display panel.

The photocatalyst thin film may include at least one of a titanium oxide (TiO2) thin film and a nitrogen-doped titanium oxide (TiO2-xNx) thin film.

The display apparatus may include at least one display apparatus selected from a group of a LCD apparatus, an OLED apparatus, and a quantum dot display apparatus.

The display panel may include a LCD panel having a first substrate and a second substrate, each of the first and second substrates including field generating electrodes are provided, and a liquid crystal layer interposed between the first and second substrates. The photocatalyst thin film may be formed on a surface of at least one of the first and second substrates.

The display apparatus may further include a backlight unit which directs to the LCD panel, and the photocatalyst thin film may be formed on a surface facing the backlight unit such that the light transmitted by the backlight unit is absorbed into the backlight unit.

In accordance with an aspect of another exemplary embodiment, a display apparatus includes a housing which forms an exterior of the display apparatus, a display panel coupled to the housing, and a photocatalyst thin film formed on a surface of the housing.

The photocatalyst thin film may include at least one of a titanium oxide (TiO2) thin film and a nitrogen-doped titanium oxide (TiO2-xNx) thin film.

The housing may be formed of a metal component or a plastic component.

The metal component may include an aluminum alloy component.

These and/or other exemplary aspects will become apparent and more readily appreciated from the following description of exemplary embodiments, taken in conjunction with the accompanying drawings in which:
FIG. 1 is a view illustrating a display apparatus according to an exemplary embodiment;
FIG. 2 is an exploded perspective view of the display apparatus of FIG. 1;
FIG. 3 is an exploded perspective view of a display module of FIG. 2;
FIG. 4 is a cross-sectional view illustrating an exemplary photocatalyst thin film is formed on a surface of a housing of a display apparatus;
FIG. 5 is a cross-sectional view illustrating an exemplary photocatalyst thin film is formed on a surface of a display panel of a display apparatus;
FIG. 6 is a configuration view illustrating a photocatalyst reaction process of titanium oxide;
FIG. 7 is a view illustrating the band gap energy of titanium oxide and the band gap energy of nitrogen-doped titanium oxide;
FIG. 8 is a graph illustrating light absorption rates of titanium oxide and nitrogen-doped titanium oxide;
FIGS. 9 to 12 are views illustrating a decomposition process of organic materials or bacteria on a surface of an exemplary display apparatus;
FIG. 13 is a cross-sectional view illustrating an organic light-emitting display apparatus according to another exemplary embodiment;
FIG. 14 is a cross-sectional view illustrating a quantum dot display apparatus according to another exemplary embodiment;
FIG. 15 is a diagram illustrating an exemplary sputtering deposition apparatus for depositing a thin film on a display apparatus;
FIG. 16 is a flowchart for describing a method of manufacturing a display apparatus according to an exemplary embodiment; and
FIG. 17 is a flowchart for describing a method of manufacturing a display apparatus according to another exemplary embodiment.

Embodiments described in this specification and configurations illustrated in the drawings are merely exemplary, and various modifications thereto and substitutions therein are also contemplated.

Hereinafter, exemplary embodiments of a display apparatus will be described in detail with reference to the accompanying drawings.

A display apparatus is an apparatus which is configured to display images. For example, a display apparatus may be a liquid crystal display (LCD) apparatus, a quantum dot display apparatus, an organic light-emitting display (OLED) apparatus, a vacuum fluorescent display apparatus, an electroluminescent device, a plasma display apparatus, etc. In this specification, it should be understood that the term "display apparatus" may mean any of these display apparatuses, or the like, as would be understood by one of skill in the art.

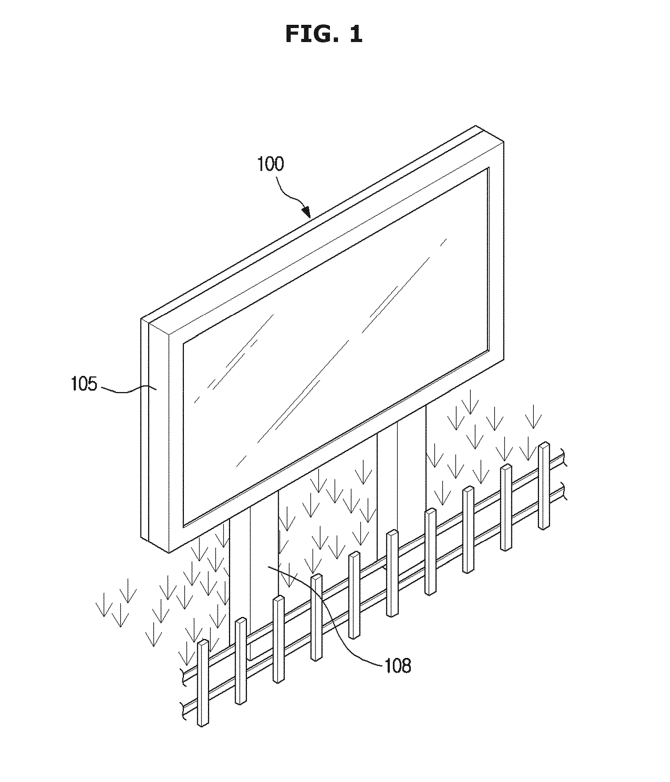

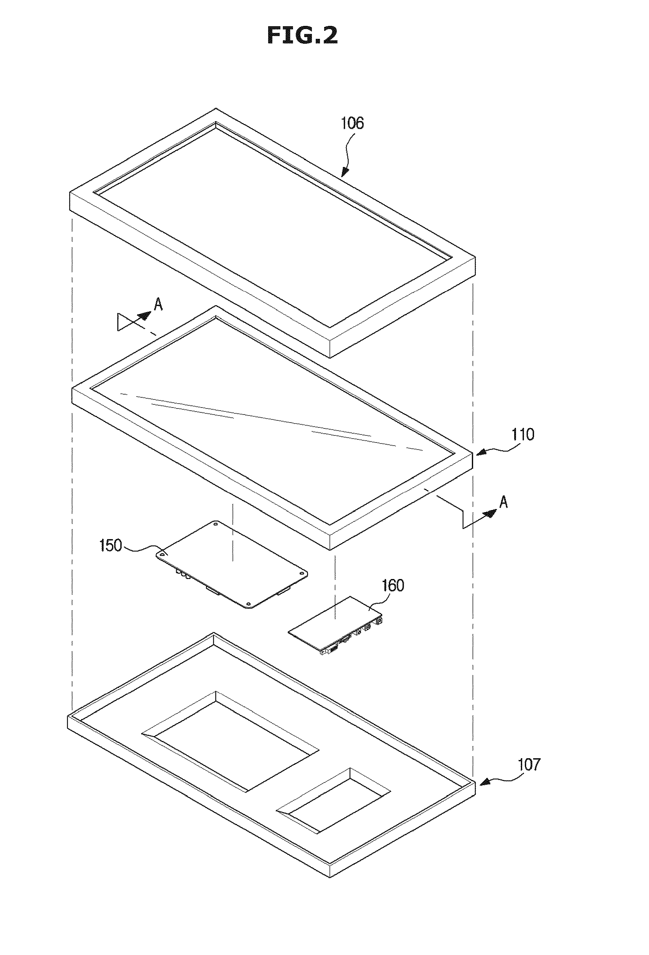

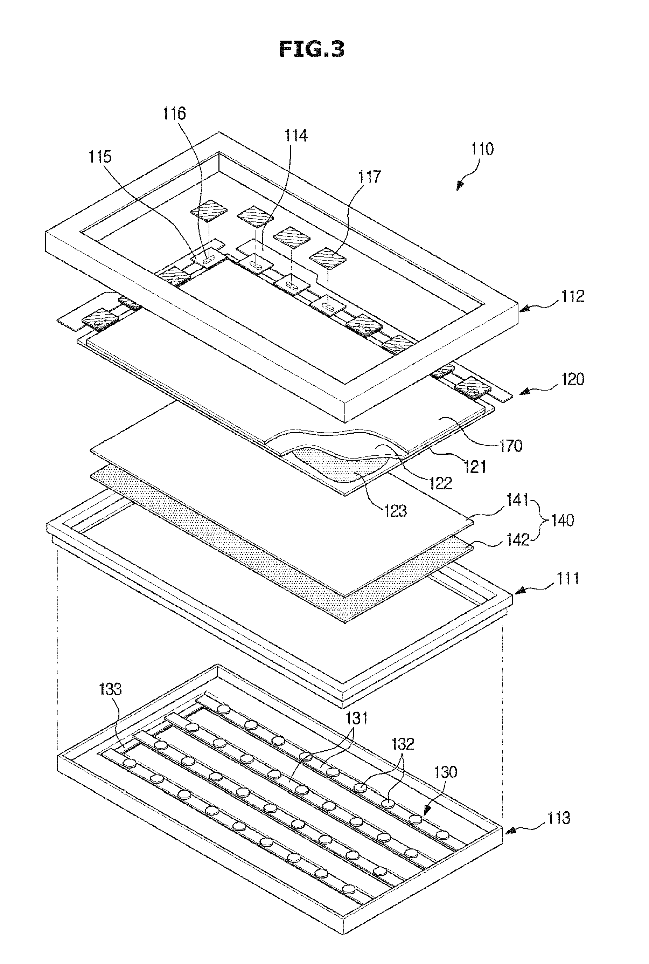

Hereinafter, an embodiment will be described using an LCD apparatus as an example. FIG. 1 is a view illustrating a display apparatus 100 according to an exemplary embodiment. FIG. 2 is an exploded perspective view of the display apparatus 100 of FIG. 1. FIG. 3 is an exploded perspective view of a display module 110 of FIG. 2.

When the display apparatus 100 is included in a monitor, a television, a tablet, a large format display (LFD), or the like, the display apparatus 100 displays images by interworking with the unique functions of the device within which it is included, and when the display apparatus 100 is included in a home appliance such as a refrigerator, an air conditioner, or the like, the display apparatus 100 displays images by interworking with the unique functions and add-on functions of the device within which it is included. In FIGS. 1 to 3, a display apparatus used as an outdoor display for advertising is illustrated as an example of one of various display apparatuses 100.

Referring to FIGS. 1 to 3, the display apparatus 100 according to this exemplary embodiment may include a housing 105 which forms an exterior of the display apparatus, and a display module 110 which is accommodated within the housing 105 and which displays images. According to one or more exemplary embodiments, a power source board 150 for supplying power, and a signal processing board 160, provided with various input terminals, which processes an external image signal to transfer to a display panel 120, to be described below, may be disposed at the rear of the display module 110.

The housing 105 forms the exterior of the display apparatus 100. In some exemplary embodiments, the housing 105 may be configured to accommodate therewithin parts such as the display panel 120 and the like. In addition, the housing 105 may include one or more accessories of the housing 105. such an accessory may include a part of the housing 105 which forms the exterior thereof, such as a bezel unit, a stand, or the like.

The housing 105 may include cases 106 and 107, which form the exterior of the display apparatus 100, and supports 108 which support the cases 106 and 107. The cases 106 and 107 may include a front case 106 and a rear case 107, coupled to each other in forward and backward directions, and the display module 110 may be installed between the front case 106 and the rear case 107. In FIGS. 1 to 3, the case in which the housing 105 is provided by assembling the front case 106 and the rear case 107 is illustrated as an example, but is not limited thereto. The housing 105 may be provided as one unitary body. Hereinafter, a case in which the housing 105 is provided by assembling the front case 106 and the rear case 107 will be described as an example for convenience of description.

The housing 105 may be formed of at least one selected from a group of a metal component and a plastic component. When the housing 105 is formed of a metal component, the housing 105 may include an aluminum alloy component. When the housing 105 is formed of a plastic component, the housing 105 may be manufactured by injection molding a plastic material. In some embodiments, the housing 105 may also include both a metal component and a plastic component, and/or may include a modification thereof, as would be understood by one of skill in the art.

A photocatalyst thin film may be formed on the housing 105. More specifically, the photocatalyst thin film may be formed one or more surfaces of the front case 106 and the rear case 107. In one or more exemplary embodiments, the photocatalyst thin film may also be formed on one or more surfaces of the support 108.

The photocatalyst thin film formed on the housing 105 may be formed to have any of a variety of different thicknesses. The thickness of the photocatalyst thin film have a thickness determined to be within a range which maintains a desired color of the housing 105.

For example, a color layer may be formed on a surface of the housing 105, and the photocatalyst thin film may be formed on an outer surface of the color layer. In this case, when the photocatalyst thin film is too thick, the color of the color layer formed on the surface of the housing 105 may be obscured due to the photocatalyst material included in the photocatalyst thin film. Thus, the thickness of the photocatalyst thin film be adjusted to be within a range which does not obscure the color of any layer therebelow. Alternately, the photocatalyst thin film may be omitted from the outer surface of the housing 105.

The display module 110 may include the display panel 120, which displays images, and a backlight unit 130, which projects light onto the display panel 120.

According to one or more exemplary embodiments, the display module 110 may include a molded interconnect device (MID) mold 111, which supports the display panel 120 and the backlight unit 130 such that they are spaced apart from each other; a top chassis 112 and a bottom chassis 113 which are disposed above the display panel 120 and under the backlight unit 130, respectively; a driving printed circuit board 114 which supplies a driving signal to the display panel 120; a flexible circuit film 115 which electrically connects the display panel 120 to the driving printed circuit board 114; a driving chip 116 mounted on a first surface of the driving printed circuit board 114; and a heat dissipation unit 117, which is disposed on a second surface of the flexible circuit film, opposite the first surface, and which dissipates heat generated by the driving chip 116.

The display panel 120 may be an LCD panel utilizing liquid crystals. More specifically, the display panel 120 may include two substrates 121 and 122 in which field generating electrodes are provided, and a liquid crystal layer 123 interposed between the two substrates 121 and 122. Here, the two substrates 121 and 122 may include a thin film transistor (TFT) substrate 121, in which TFTs are formed, and a color filter substrate 122 facing the TFT substrate 121.

The TFT substrate 121 is a transparent glass substrate in which the TFTs, which are switching devices, are formed in a matrix form. A data line may be connected to a source terminal of a TFT, a gate line may be connected to a gate terminal of the TFT, and a pixel electrode made of a transparent conductive material may be connected to a drain terminal of the TFT.

The color filter substrate 122 is disposed such that it is spaced apart from the TFT substrate by a predetermined interval and such that it is opposite the TFT substrate. The color filter substrate 122 is a substrate on which a red pixel, a green pixel, and a blue pixel, which are color pixels which transmit light of a predetermined color, are formed by a thin film process. In some exemplary embodiments, a common electrode (not illustrated) made of a transparent conductive material may be formed on a front surface of the color filter substrate 122.

In the display panel 120 having this configuration, when power is applied to the gate terminal of a TFT, an electric field is formed between the pixel electrode and the common electrode, and an orientation of liquid crystals included in the liquid crystal layer 123 provided between the TFT substrate 121 and the color filter substrate 122 is changed by the electric field. Thus, the display panel 120 forms an image by adjusting the orientation of the liquid crystals included in the liquid crystal layer 123. However, since the display panel 120 may not emit light itself, the display panel 120 may be supplied with light from the backlight unit 130, located on a rear surface of the display panel 120, to display the image.

A photocatalyst thin film may be formed on the display panel 120. More specifically, the photocatalyst thin film may be formed on a surface of at least one of the TFT substrate 121 and the color filter substrate 122 of the display panel 120. According to an exemplary embodiment, the photocatalyst thin film may be formed on a surface of the TFT substrate 121 or the color filter substrate facing the backlight unit 130, such that light emitted from the backlight unit 130 is absorbed by the photocatalyst thin film. A film (not illustrated) or a cover glass (not illustrated) for protecting the display panel 120 may also be disposed on a surface of the display panel 120. In this case, the photocatalyst thin film may be formed on a surface of the film or cover glass.

The photocatalyst thin film formed on the display panel 120 may be formed to have any of a variety of different thicknesses. Although the thickness is not particularly limited, the photocatalyst thin film may have a thickness within a range which does not obscure the sharpness of an image displayed by the display panel 120. The photocatalyst thin film may have the thickness in a range of 50 nm to 1 µm, but the thickness thereof is not limited thereto. The photocatalyst thin film will be described in detail below.

The backlight unit 130, which is disposed on an inner side of the housing 105, to provide light, may efficiently transmit light, generated by a light source, to the display panel 120 via optical compensation. According to an exemplary embodiment, the backlight unit 130 may include at least one printed circuit board 131 in which a conductive pattern is formed and which is disposed at the rear of the display panel 120, and one or more light emitting diodes (LEDs) 132 mounted on a front surface of the printed circuit board 131, such that the one or more LEDs face a rear surface of the display panel 120.

The printed circuit board 131 extend in a length direction, and a plurality of printed circuit boards 131 may be disposed such that they spaced apart and parallel to each other. In this case, a connection board 133, which connects ends of the printed circuit boards 131 to each other, may be disposed at the ends of the printed circuit boards, and the printed circuit boards 131 may be interworked and may operate via the connection board 133.

A plurality of LEDs 132 may be provided, and arrayed along a longitudinal (length) direction of the printed circuit board 131. According to an exemplary embodiment, a lens which diffuses light generated by the LED 132 may be disposed on the LED 132. A plurality of lenses may be provided and disposed on the plurality of LEDs 132, respectively.

Optical sheets 140, for improving an optical characteristic of light emitted from the backlight unit 130, may be provided between the display panel 120 and the backlight unit 130. The optical sheets 140 diffuse and collect the light emitted from the backlight unit 130, and transmit the light to the display panel 120. The optical sheets 140 may include one or more diffusion sheets 141 and light collecting sheets 142. The diffusion sheet 141 serves to improve the uniformity of brightness by diffusing the light emitted from the backlight unit 130, and the light collecting sheet 142 serves to supply light uniformly to the display panel 120 by collecting the light diffused by the diffusion sheet 141.

A single diffusion sheet 141 may be provided, and the light collecting sheet 142 may include a first light collecting sheet and a second light collecting sheet which are oriented perpendicular to each other, in an x-axis direction and a y-axis direction, respectively. The light collecting sheet 142 may improve a coherence of light by refracting light in the x-axis and the y-axis directions.

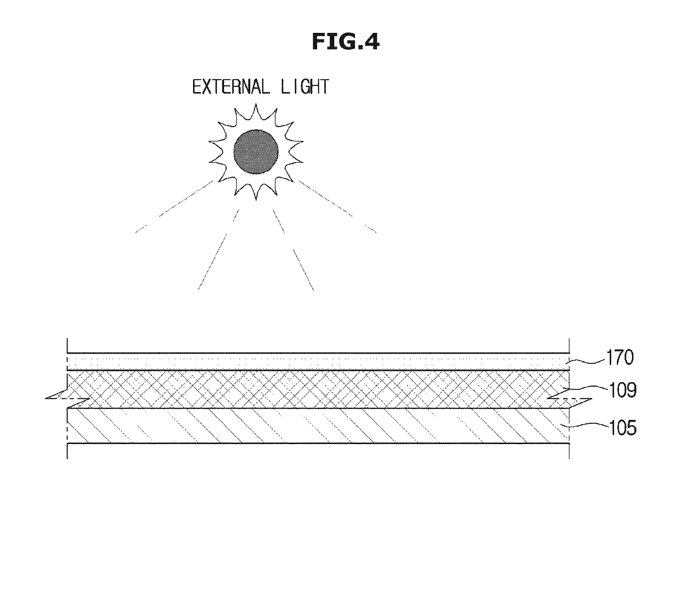

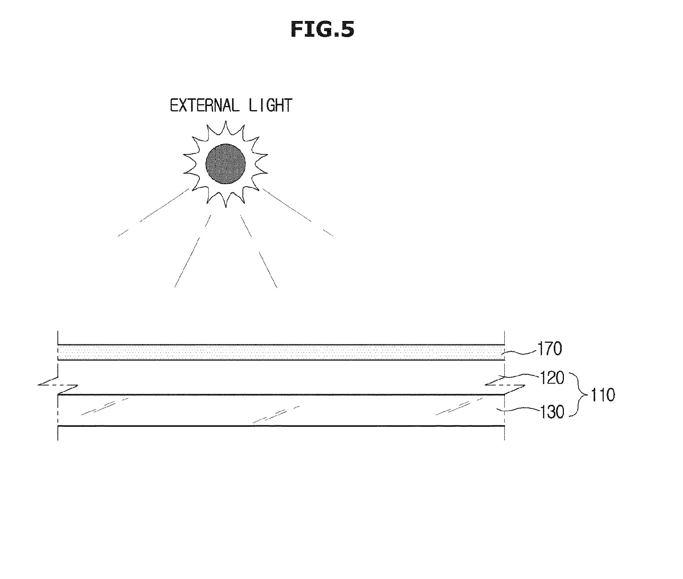

Hereinafter, the photocatalyst thin film of the display apparatus 100 according to the present exemplary embodiment will be described in more detail below. FIGS. 4 and 5 are cross-sectional views illustrating the display apparatus 100 in which a photocatalyst thin film 170 is formed. More specifically, FIG. 4 is a cross-sectional view illustrating an exemplary aspect in which the photocatalyst thin film 170 is formed on a surface of the housing 105 of the display apparatus 100, and FIG. 5 is a cross-sectional view illustrating an exemplary aspect in which the photocatalyst thin film 170 is formed on a surface of the display panel 120 of the display apparatus 100.

Referring to FIG. 4, a color layer 109, for implementing the sense of color, may be formed on the housing 105 of the display apparatus 100 according to an exemplary embodiment, and the photocatalyst thin film 170, for providing a photocatalyst activity, may be formed on an upper surface of the color layer 109. Alternately, the color layer 109 may be omitted. In such a case, the photocatalyst thin film 170 may be directly coupled to a surface of the housing 105. In addition, the photocatalyst thin film 170 may be formed on an entire surface of the color layer 109 or the housing 105 or may be formed on only a part of a surface of the color layer 109 or the housing 105.

The photocatalyst thin film 170 may absorb external light to proceed with a photocatalyst reaction, and the external light may include sunlight, light output from a fluorescent light, etc.

Referring to FIG. 5, the photocatalyst thin film 170 may be formed on the display panel 120 of the display apparatus 100 according to an exemplary embodiment, and the photocatalyst thin film 170 may be formed on an entire surface of the display panel or on only a part of a surface of the display panel 120. In the example of FIG. 5, the photocatalyst thin film 170 is formed on the surface of the display panel 120, but this is merely exemplary, and the photocatalyst thin film 170 is not limited thereto.

The photocatalyst thin film 170 according to the present exemplary embodiment may absorb light emitted from the backlight unit 130 and external light to cause the photocatalyst reaction. This is enabled by the positioning of the backlight unit 130 on a rear surface of the display panel 120. It should also be noted that the light emitted from the backlight unit 130 can also be used to display an image, even during the photocatalyst reaction, unlike in the case of FIG. 4.

The photocatalyst thin film 170 may include a photocatalyst material having a photocatalyst activity. In general, a photocatalyst, which is a catalyst which uses light as an energy source, may perform a self-cleaning function, a sterilization function, or the like by accelerating photoreaction.

When the photocatalyst material having a photocatalyst activity absorbs light having an energy corresponding to a band gap between a valance band and a conduction band, electrons (e-) present in the valance band are transitioned to the conduction band. The electrons transitioning to the conduction band try to move to a material adsorbed onto the photocatalyst material. Thus, when the material is adsorbed onto the photocatalyst material, the corresponding material may be recovered by the electrons. When the electrons present in the valance band are transitioned to the conduction band, holes (h+) are generated in the valance band. The holes generated in the corresponding valance band take electrons from a material adsorbed onto a surface of the photocatalyst material. Thus, when the material is adsorbed onto the surface of the photocatalyst material, electrons of the corresponding material move to the holes, and thus the corresponding material may be oxidized.

The photocatalyst material capable of effectively performing the photocatalyst reaction may be, for example, titanium oxide (TiO2). In some exemplary embodiments, nitrogen-doped titanium oxide (TiO2-xNx) may be used.

According to an exemplary embodiment, a material such as titanium oxide (TiO2), nitrogen-doped titanium oxide (TiO2-xNx), or the like may be formed by a deposition process, and more specifically, by a reactive sputtering process. Materials such as titanium oxide (TiO2), nitrogen-doped titanium oxide (TiO2-xNx), or the like may be easily deposited on a glass surface or a plastic surface when a predetermined deposition condition is satisfied. The detailed deposition condition will be described below in relevant part.

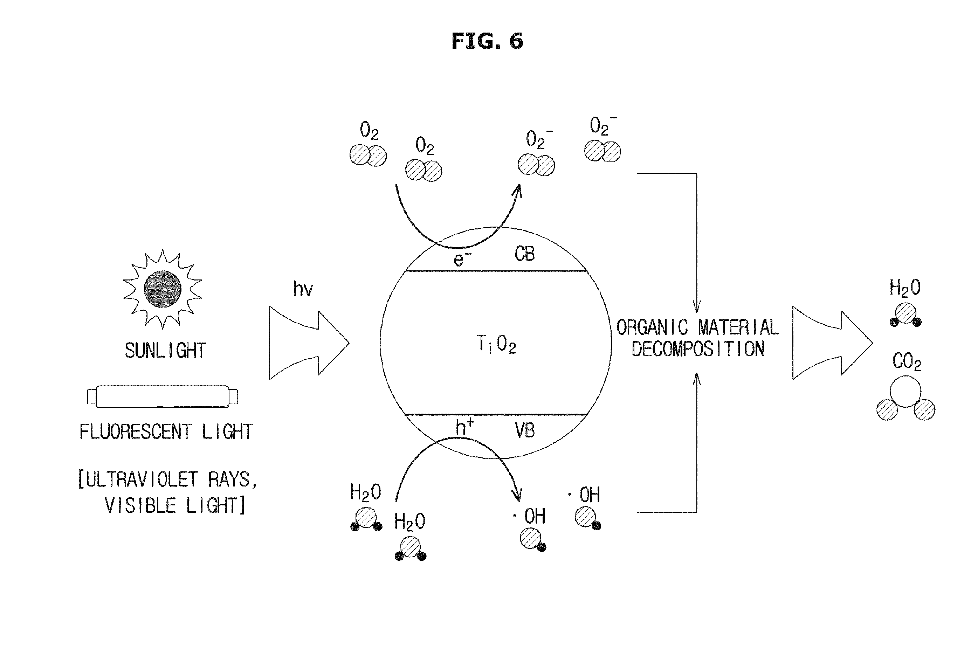

Hereinafter, a principle of the photocatalyst reaction in the photocatalyst thin film 170 will be described in detail. FIG. 6 is a configuration view illustrating a photocatalyst reaction process of titanium oxide (TiO2).

Referring to FIG. 6, when titanium oxide absorbs light having an energy corresponding to a band gap between a valance band and a conduction band of the titanium oxide, electrons present in the valance band of the titanium oxide are transitioned to the conduction band, and the transitioned electrons generate a superoxide anion (O2-) by recovering oxygen (O2) in the air. As a result of the transfer of the electrons, holes are generated in the valance band, and the generated holes generate a hydroxyl radical (•OH) by oxidizing water (H2O) adsorbed onto a surface of the titanium oxide.

Since the hydroxyl radical has a very strong oxidizing power, when an organic material or the like adsorbs onto the surface of the titanium oxide, the corresponding organic material or the like decomposes by an action of the hydroxyl radical, and finally, may decompose to water (H2O), carbon dioxide (CO2), etc.

The titanium oxide having the photocatalyst activity should absorb light energy of about 3.2 eV in order to excite the electrons located in the valance band into the conduction band. When this light energy is converted into a wavelength of light, a wavelength of about 380 nm is obtained. That is, when titanium oxide is used as the only photocatalyst material, it may be difficult to implement the photocatalyst activity using visible light (having a wavelength in a range of about 400 nm to 800 nm). Thus, in one or more exemplary embodiments, a photocatalyst material on which a metallic material or a non-metallic material is doped may be used to efficiently use the visible light as an energy source for the photocatalyst activity.

When the photocatalyst material in which the metallic material or the non-metallic material is doped is used, the band gap energy may be reduced compared to a case in which the titanium oxide is used alone. As a result, photocatalyst efficiency can be improved.

As the metallic material, most transition elements may be used. For example, ruthenium (Ru), silver (Ag), platinum (Pt), copper (Cu), molybdenum (Mo), niobium (Nb), vanadium (V), iron (Fe), cobalt (Co), nickel (Ni), chrome (Cr), manganese (Mn), or the like may be used. As the non-metallic material, carbon (C), sulfur (S), nitrogen (N), phosphate (P), boron (B), iodine (I), fluorine (F), or the like may be used. In addition to the metallic material or the non-metallic material, the band gap energy may be reduced using a composite material.

Hereinafter, a band gap energy difference, depending on the photocatalyst material, and an improvement of the efficiency of the photocatalyst material, depending on the band gap energy difference, will be described in detail. For convenience, titanium oxide (TiO2) is used as an example of a photocatalyst material having a single component and nitrogen-doped titanium oxide (TiO2-xNx) is used as an example of a photocatalyst material having a composite component.

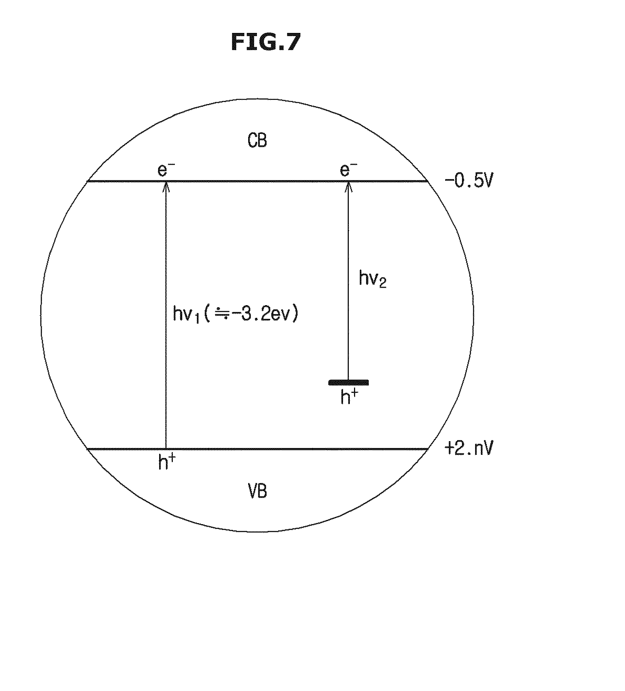

FIG. 7 is a view illustrating the band gap energy of titanium oxide (TiO2) and the band gap energy of nitrogen-doped titanium oxide (TiO2-xNx).

Referring to FIG. 7, the band gap energy hv1 of the titanium oxide (TiO2) is a relatively high band gap energy of about -3.2 eV. The titanium oxide may mainly absorb light in an ultraviolet range due to the high band gap energy, and thus, it is difficult to use visible light, having a relatively low energy, as described above.

About 45 % of sunlight has a wavelength in the range of visible light, and most light output by an LED light source (which may be used as the backlight unit 130) also has a wavelength in the range of visible light. Thus, in the present exemplary embodiment, a nitrogen material is doped into the titanium oxide, and thus the band gap energy of the photocatalyst material may be lowered. When the band gap energy of the photocatalyst material is lowered, visible light may also be used, and thus light efficiency may be improved. As illustrated in FIG. 7, when nitrogen is doped, energy in the valance band is increased and thus the band gap may be reduced to hv2. Thus, the efficiency of the photocatalyst may be improved by utilizing the absorption of both visible light and ultraviolet light.

However, when the band gap is reduced more than necessary, the electrons excited into the conduction band and the holes formed in the valance band may be recombined. When the electrons and the holes are recombined, heat may be generated. In this case, the heating of the display apparatus 100 may occur. Therefore, the electrons should be easily excited, and at the same time, the band gap should be appropriately adjusted to avoid the recombination of the electrons and the holes.

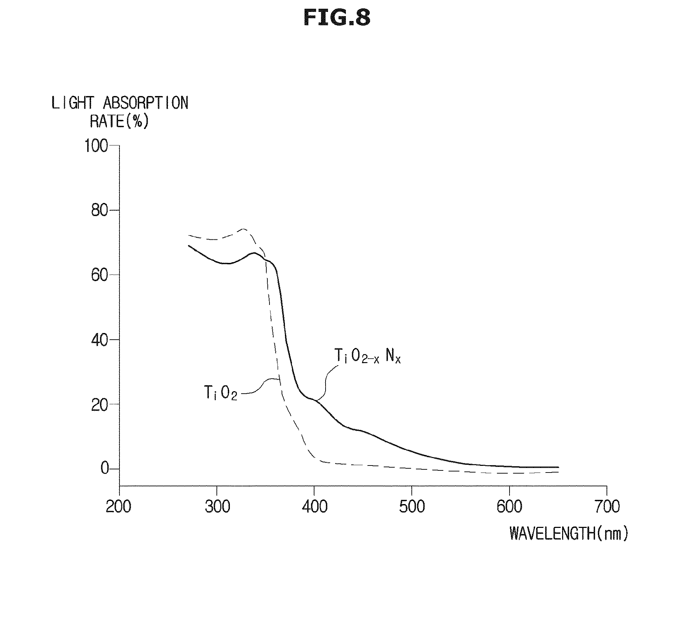

FIG. 8 is a graph illustrating light absorption rates of titanium oxide (TiO2) and nitrogen-doped titanium oxide (TiO2-xNx). The horizontal axis of FIG. 8 indicates the wavelength of light and the vertical axis of FIG. 8 indicates the absorption rate of the light.

Referring to FIG. 8, it may be seen that the titanium oxide has a high light absorption rate for wavelengths of 400 nm or less, while, on the other hand, the nitrogen-doped titanium oxide has a higher light absorption rate than the titanium oxide for wavelengths of 400 nm or more. That is, when a nitrogen-doped titanium oxide material is used as the photocatalyst material, it may be seen that light having a greater wavelength may be used according to the reduction of the band gap energy. As a result, it may be seen that the efficiency of the photocatalyst material is improved.

Hereinafter, a self-cleaning process and a sterilization process of the surface of the display panel 120 according to an exemplary embodiment will be described in more detail below.

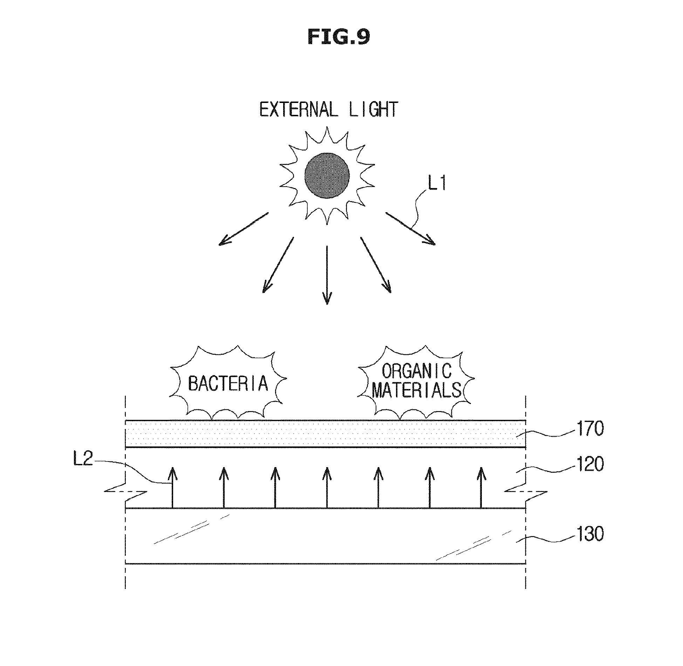

FIGS. 9 to 12 are views illustrating a decomposition process of organic materials or bacteria on a surface of a display apparatus 100. The decomposition process of the organic materials or the bacteria using a surface of a display panel 120 will be described as an example for convenience.

FIG. 9 is a view illustrating an exemplary embodiment in which light is incident on the display panel 120 on which a titanium oxide photocatalyst thin film 170 is deposited.

As illustrated in FIG. 9, the surface of the display panel 120 may be contaminated by bacteria and organic materials. When light is incident on the display panel 120, the photocatalyst thin film 170 absorbs the light. Here, the light incident on the photocatalyst thin film 170 may include light having an energy equaling at least that of the band gap of titanium oxide.

The light incident on the photocatalyst thin film 170 may be external light L1 from sunlight or a fluorescent light source, and internal light L2 output from the backlight unit 130. For example, while the display apparatus 100 operates, the light L2 emitted from the backlight unit 130 may be provided to the photocatalyst thin film 170, along with the external light L1, and while the display apparatus 100 is not in operation, only the external light L1 is provided to the photocatalyst thin film 170. The incident light L1 and L2 may include visible light and ultraviolet light, respectively.

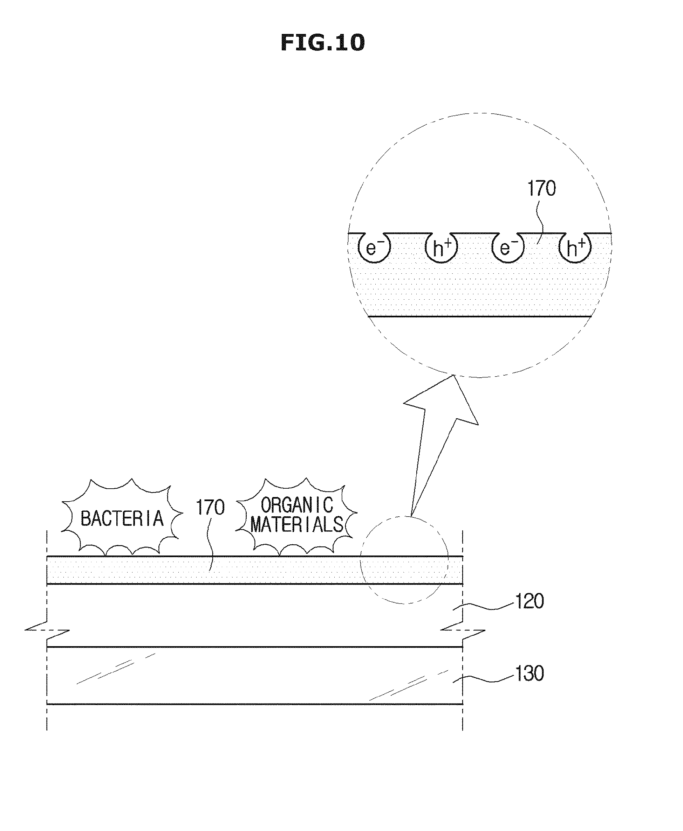

FIG. 10 is a view illustrating electrons and holes formed in the photocatalyst thin film 170, according to an exemplary embodiment.

When light in a wavelength having an energy equaling at least that of a band gap of the material of the photocatalyst thin film 170 is emitted to the photocatalyst thin film 170, electrons on the surface of the titanium oxide may be excited from a valance band to a conduction band as illustrated in FIG. 10. At the same time, holes may be generated in the valance band, and the generated electrons and holes are diffused and moved onto the surface of the titanium oxide.

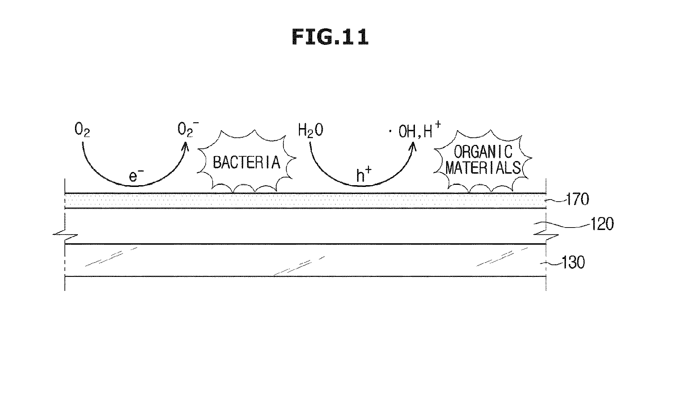

FIG. 11 is a view illustrating electrons and holes, which are present on an inside or a surface of the photocatalyst thin film 170, reacting with oxygen and water in the air, respectively, according to an exemplary embodiment.

As illustrated in FIG. 11, electrons and holes, which are present on the inside or a surface of the photocatalyst thin film 170, may react with oxygen and water in the air, respectively. Hereinafter, Reaction Formula 1 represents a process in which the superoxide anion (O2-) is generated by the reaction of the oxygen and the electrons, and Reaction Formula 2 represents a process in which the hydroxyl radical (•OH) is generated by the reaction of the water and the holes.

Reaction Formula 1: O2 + e- → O2-

Reaction Formula 2: H2O + h+ → •OH + H+

According to Reaction Formula 1 and Reaction Formula 2, the superoxide anion (O2-), the hydroxyl radical (•OH), and the like are present on the surface of the photocatalyst thin film 170.

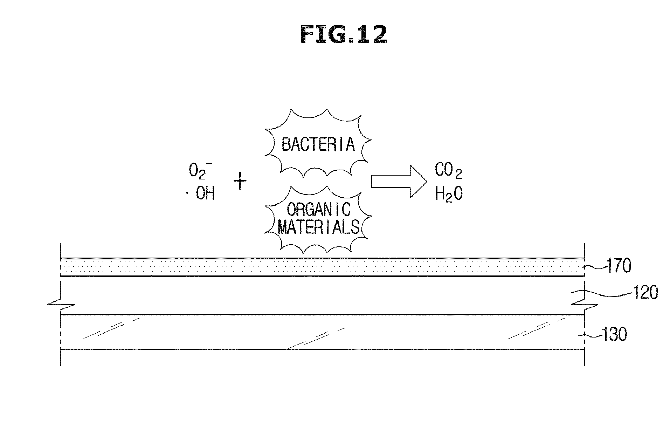

As illustrated in FIG. 12, active species such as the superoxide anion (O2-) and the hydroxyl radical (•OH) may decompose organic materials or bacteria which are present on the surface of the photocatalyst thin film 170. As a result of the decomposition, carbon dioxide (CO2) or water (H2O) may be generated.

In the above-described exemplary embodiment, an LCD apparatus 100 is used as an example. Next, a display apparatus according to another exemplary embodiment will be described.

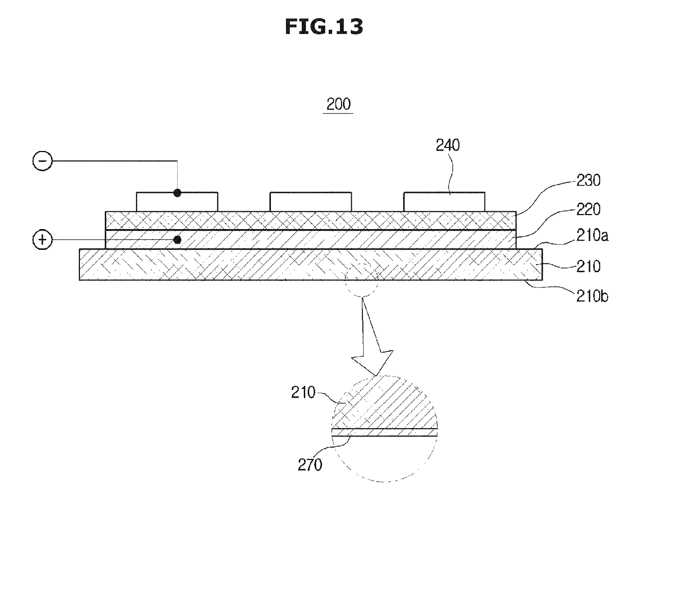

The display apparatus according to another exemplary embodiment may include an OLED apparatus. FIG. 13 is a cross-sectional view illustrating an OLED apparatus 200 according to another exemplary embodiment.

Referring to FIG. 13, the OLED apparatus 200 may include a substrate 210; an anode layer 220 stacked on the substrate 210; an organic material layer 230, which is stacked on the anode layer 220 and which emits light; and a cathode layer 240, which is stacked on the organic material layer 230 and which reflects light generated in the organic material layer 230 toward the substrate 210.

The substrate 210 may be configured to support the display apparatus 200. The substrate 210 may be made of a synthetic resin and may include a catalytic component to increase the interference of light and a diffraction rate. According to this exemplary embodiment, the substrate 210 may be formed of a material such as polyethylene terephthalate (PET), polyethylene naphthalate (PEN), polyethersulfone (PES), or the like, which has excellent transmittance, surface resistance, mechanical characteristics, and thermal expansion coefficient. However, the substrate 210 is not limited thereto, and may be formed of another plastic material, a glass material, a foil material, or the like.

A photocatalyst thin film 270 may be formed on a surface of the substrate 210. The photocatalyst thin film 270 may include titanium oxide (TiO2), nitrogen-doped titanium oxide (TiO2-xNx), or the like. The photocatalyst thin film 270 may be provided on at least one of an upper surface 210a of the substrate 210 and a lower surface 210b of the substrate 210. The upper surface 210a of the substrate 210 may be defined as a surface on which the anode layer 220 is stacked, and the lower surface 210b of the substrate 210 may be a surface opposite to the surface on which the anode layer 220 is stacked.

The photocatalyst thin film 270 may be provided to add a self-cleaning function or a sterilization function to the display apparatus 200. Hereinafter, any repetitive description of the above-described photocatalyst thin film 170 will be omitted.

When power is applied, the anode layer 220 may generate holes, and the generated holes may be transferred to the organic material layer 230. A hole transport layer (not illustrated) for transferring the holes may be provided between the anode layer 220 and the organic material layer 230. Alternately, when power is applied, the cathode layer 240 may generate electrons, and the generated electrons may be transferred to the organic material layer 230. According to one or more exemplary embodiments, an electron transport layer (not illustrated) for transferring the electrons may be provided between the cathode layer 240 and the organic material layer 230.

The organic material layer 230 may be provided between the hole transport layer and the electron transport layer, and light may be emitted by the recombination of the holes generated in the anode layer 220 and the electrons generated in the cathode layer 240. More specifically, when the holes generated in the anode layer 220 and the electrons generated in the cathode layer 24 are recombined, excitons may be formed, and at the same time, light may be emitted. That is, the organic material layer 230 may serve as a light emission layer. The organic material layer 230 may be formed of an organic compound including carbon (C), hydrogen (H), oxygen (O), phosphorus (P), sulfur (S), and the like, but is not limited thereto.

As described above, the OLED apparatus 200 is used as an example. Next, a display apparatus according to still another exemplary embodiment will be described.

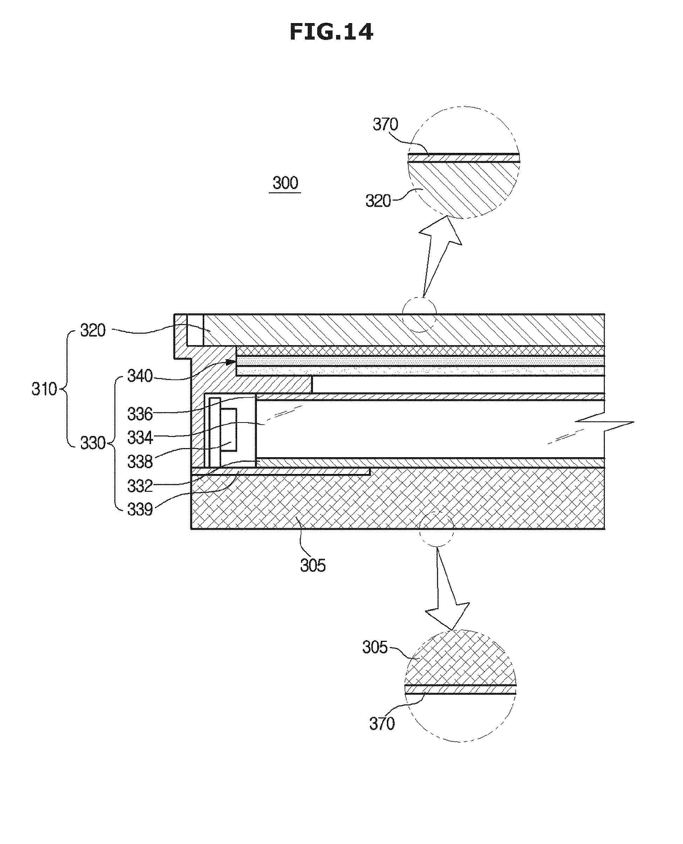

The display apparatus according to another exemplary embodiment may include a quantum dot display apparatus. FIG. 14 is a cross-sectional view illustrating a quantum dot display apparatus 300 according to another exemplary embodiment. Referring to FIG. 14, the quantum dot display apparatus 300 may include a housing 305 which forms an exterior of the quantum dot display apparatus, and a display module 310 which is accommodated within the housing 305 and which displays images.

The housing 305 is substantially the same as the housing 105 according to FIGS. 1 to 3. That is, a photocatalyst thin film 370 may also be formed on a surface of the housing 305 according to the present exemplary embodiment. Hereinafter, any repetitive description will be omitted.

Referring to FIG. 14, the display module 310 according to the present exemplary embodiment may include a display panel 320 and a backlight unit 330.

The components of the LCD panel 120 illustrated in FIG. 1 to 3 may be applied to the display panel 320. In addition, as illustrated in FIG. 5, the photocatalyst thin film 370 may be formed on a surface of the display panel 320. Hereinafter, any repetitive description will be omitted.

The backlight unit 330 is disposed on an inner side of the housing 305 to output light. The backlight unit 330 according to the present exemplary embodiment may include a reflective sheet 332 disposed on an inner bottom surface of the housing 305, a light guide plate 334 disposed on an upper surface of the reflective sheet 332, a quantum dot sheet 336 disposed on the light guide plate 334, a plurality of optical sheets 340 disposed on the quantum dot sheet 336, a light emitting diode 338 disposed on a side surface of the light guide plate 334, and a printed circuit board 339 on which the light emitting diode 338 is mounted.

The reflective sheet 332 is disposed under the light guide plate 334 to reflect light, output from the light emitting diode 338, toward the light guide plate 334. More specifically, the reflective sheet 332 serves to reflect light, previously refracted downward from the light guide plate 334, back to the light guide plate 334, and thus the efficiency of the display module can be improved.

The light guide plate 334 is disposed on the reflective sheet 332, receives the light emitted from the light emitting diode 338, and directs the light toward the quantum dot sheet 336 via reflection, refraction, scattering, etc. The light guide plate 334 may include a light-diffusing material to assist in light transmission, and a predetermined pattern, a groove, or the like for scattering incident light may be formed on a rear surface of the light guide plate 334.

The quantum dot sheet 336 may include quantum dots processed in a sheet form. Quantum dots are semiconductor crystals into which self-emitted quanta are injected in nanometer units when a current flows. The quantum dot sheet 336 according to the present exemplary embodiment may be configured to transmit at least a portion of blue light incident on an incident surface of the light guide plate 334, and to convert the remaining blue light into green light and red light, and to convert white light to reach the display panel 320.

The quantum dot sheet 336 may include a resin layer (not illustrated) in which the quantum dots are dispersed, and a protection layer (not illustrated) and a coating layer (not illustrated), which surround the resin layer. The plurality of quantum dots which convert the wavelength of incident light are sprayed on the resin layer, which may be made of an acrylate polymer resin material to transmit the incident light without loss. The quantum dot sheet 336 may adjust an amount of light incident on the display panel 320 in each wavelength to implement a high color reproducibility.

The optical sheet 340 is disposed between the display panel and the quantum dot sheet 336, diffuses the light output from the quantum dot sheet 336, collects the light, and transmits the light to the display panel 320. The components of FIG. 3 may be applied to the optical sheet 340. Hereinafter, repetitive description will be omitted.

A plurality of light emitting diodes 338 may be arranged such that they are spaced apart from each other by a predetermined interval. Each of the light emitting diodes 338 may include a point light source, and may be disposed on a side surface of the light guide plate 334 to provide light.

It should be noted that the quantum dot display apparatus 300 is not limited to the specific description above. Various forms in which the light source includes quantum dots, such as a quantum dot sheet 336 disposed on the side surface of the light guide plate 334, or the like may be applied.

As described above, various embodiments of the display apparatuses 100, 200, and 300 are described. Hereinafter, methods of manufacturing the display apparatuses 100, 200, and 300, and more specifically, manufacturing methods in which the photocatalyst thin films 170, 270, and 370 are formed on surfaces of the display apparatuses 100, 200, and 300, will be described. The display apparatus 100 according to FIGS. 1 to 3 will be described as an example, in order to facilitate understanding, and the same contents as a method of forming the photocatalyst thin film 170 to be described below may be applied to methods of manufacturing the OLED apparatus 200 and the quantum dot display apparatus 300.

As a method of forming the photocatalyst thin film 170 on the surface of the display apparatus 100, a sputtering method may be applied.

A sputtering method, which is a physical vapor deposition method, is a method in which an inert gas is ionized, the ionized inert gas is collided with a solid sample in a vacuum chamber, and atoms are ejected from the solid sample by energy generated during the collision. The sputtering method may be used for forming a metal layer of a thin film in the manufacture of a semiconductor, a display device, or the like, or depositing a metal oxide layer. Hereinafter, argon (Ar) will be described as an example of the inert gas ionized in the vacuum chamber. However, the inert gas is not limited thereto, and another inert gas may be used or argon (Ar) may be used by being mixed with one or more other inert gases.

To aid in understanding, first, a configuration of a sputtering deposition apparatus will be described, and then a method of manufacturing the display apparatus 100 by the sputtering deposition apparatus will be described.

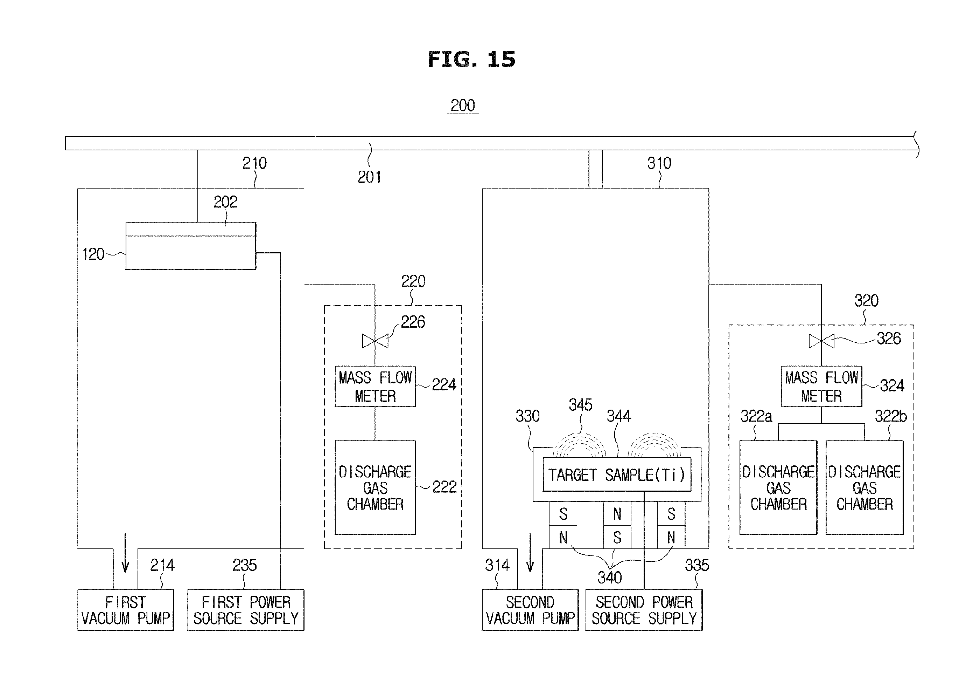

FIG. 15 is a diagram illustrating an example of a sputtering deposition apparatus 200 for depositing a thin film on the display apparatus 100, according to an exemplary embodiment.

Referring to FIG. 15, the sputtering deposition apparatus 200 may include vacuum chambers 210 and 310, vacuum pumps 214 and 314, gas supply systems 220 and 320, a rail 201, a gun 330, a target sample 344, and a plurality of magnetrons 340.

The vacuum chambers 210 and 310 are chambers in which a deposition process is performed, and the insides thereof may be maintained in a vacuum state by the vacuum pumps 214 and 314, respectively. The first vacuum chamber 210 may be a chamber in which a plasma process is performed, and the second vacuum chamber 310 may be a chamber in which the deposition process of the photocatalyst thin film 170 (see FIG. 5) is performed. In some embodiments, the first vacuum chamber 210 may be omitted.

The vacuum pumps 214 and 314 may be provided on side surfaces of the vacuum chambers 210 and 310, respectively, and may maintain the vacuum states of the vacuum chambers 210 and 310.

The gas supply systems 220 and 320 are provided on side walls of the vacuum chambers 210 and 310, respectively, and may supply gases into the vacuum chambers 210 and 310. The gas supply systems 220 and 320 may include discharge gas chambers 222 and 322a, in which an argon gas to be ionized is stored; a reaction gas chamber 322b in which an oxygen gas is stored for performing a plasma chemical deposition process, mass flow meters 224 and 324, which connect the vacuum chambers 210 and 310 to the gas chambers 222, 322a, and 322b; and control valves 226 and 326 which control gas flow from the discharge gas chambers 222 and 322a or the reaction gas chamber 322b to the vacuum chambers 210 and 310.

According to an exemplary embodiment, nitrogen gas may be stored in the reaction gas chamber 322b with an oxygen gas to form a nitrogen-doped titanium oxide thin film.

A rail 201 may be disposed over the vacuum chambers 210 and 310 and an object to be deposited may be mounted on the rail 201. More specifically, the object may be held by the jig 202 and moved along the rail 201. The housing 105 or the display panel 120 of the display apparatus 100 may be provided as the object. Hereinafter, a case in which the display panel 120 is provided as the object will be described as an example for convenience of description.

The target sample 344 may be provided inside the second vacuum chamber 310, and may be oriented to be faces the display panel 120 when the display panel 120 is moved inside the second vacuum chamber 310 as the object. When an object being used has a curved shape, a plurality of target samples may be used. In the present exemplary embodiment, since the titanium oxide thin film 170 (see FIG. 5) will be deposited, titanium (Ti) may be used as the target sample 344. A process of supplying oxygen from the reaction gas chamber 322b is not performed, and titanium oxide (TiO2) by itself may be used as the target sample 344.

The gun 330 may be provided inside the second vacuum chamber 310. More specifically, the gun 330 may be connected to negative power through the second power source supply 335. When the second power source supply 335 supplies power to the gun 330, negative electric fields are generated, discharging is started, and then plasma may be generated while generating argon ions.

The magnetrons 340 may be provided inside the second vacuum chamber 310 and a plurality of magnetrons 340 may be installed under the target sample 344. A magnetic field 345 is formed by the magnetrons 340 and thus electrons separated from the argon gas perform a spiral movement as they simultaneously receive a force of the magnetic field 345 formed by the magnetrons 340 in the existing electric field. Since the electrons in which perform the spiral movement are trapped in the magnetic field 345 and escaping is difficult, the density of the electrons in the plasma is increased.

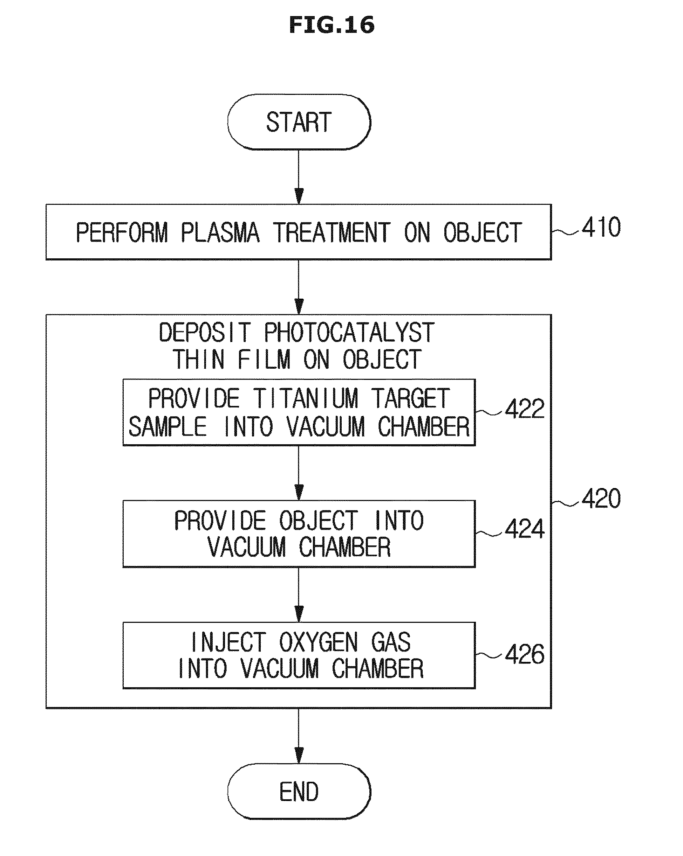

Because of this, the number of ionized argon ions is increased in the second vacuum chamber 310, the number of the argon ions colliding with the target sample 344 is also increased, and thus a thin film deposition process may be smoothly performed. Hereinafter, the thin film deposition process of the display apparatus 100 will be described in detail with reference to FIG. 15 described above. FIG. 16 is a flowchart for describing a method of manufacturing a display apparatus 100 according to an exemplary embodiment.

Referring to FIG. 16, a method of manufacturing the display apparatus 100 according to an exemplary embodiment may include performing a plasma treatment on an object (S410) and depositing a photocatalyst thin film 170 including titanium oxide on the object (S420). The depositing of the photocatalyst thin film 170 including the titanium oxide on the object (S420) may include providing a titanium target sample 344 into a vacuum chamber 310 (S422), providing the object into the vacuum chamber 310 (S424), and injecting an oxygen gas serving as a reaction gas inside the vacuum chamber 310 (S426).

The object may include at least one selected from a group of a housing 105 which forms an exterior of the display apparatus 100 and a display panel 120 coupled to the housing 105. Hereinafter, an exemplary embodiment in which the display panel 120 is provided as the object will be described.

First, the process in which the display panel 120 is provided in the first vacuum chamber 210 of a sputtering deposition apparatus 200 and a surface thereof is modified through a plasma treatment under appropriate conditions may be performed (S410). When power is supplied to a jig 202 through a first power source supply 235 and a negative electric field is generated, discharging is started in the first vacuum chamber 210 and plasma is generated. The argon gas reacts as in the following Reaction Formula 3 and thus plasma may be generated.

Reaction Formula 3 Ar → Ar+ + e-

A plasma treatment process, which is a process for more effectively depositing the photocatalyst thin film 170, may be selectively performed. According to one or more exemplary embodiments, a plasma power range may be provided within a range of 1 kw to 15 kw, but embodiments are not limited thereto.

Next, the process in which the photocatalyst thin film 170 including the titanium oxide is deposited on the object may be performed (S420).

In the manufacturing method according to the present exemplary embodiment, the titanium target sample 344 may be provided in a second vacuum chamber 310 in advance (S422). According to one or more exemplary embodiments, a titanium oxide target sample rather than the titanium target sample may be provided.

Next, the process in which the display panel 120 is provided into the second vacuum chamber 310 may be performed (S424). The display panel 120 may be fixed to the jig 202 to be provided into the second vacuum chamber 310 along the rail 201. One surface of the display panel 120, which is provided into the second vacuum chamber 310 and faces the target sample 344, may be processed by the plasma treatment.

Once the display panel 120 is moved inside the second vacuum chamber 310, the process in which an oxygen gas is injected inside the second vacuum chamber 310 may be performed (S426). More specifically, control valve 326 is controlled while the second vacuum chamber 310 is maintained in a vacuum state by the second vacuum pump 314, and then an argon gas and the oxygen gas may be injected into the second vacuum chamber 310.

In operation S426, when the titanium target sample 344 is used, the argon gas may be injected within a range of 60 sccm to 360 sccm and the oxygen gas may be injected within a range of 30 sccm to 180 sccm. According to one or more exemplary embodiments, when a titanium oxide target sample is used, only the argon gas is be injected. In such an embodiment, the argon gas may be injected within a range of 60 sccm to 360 sccm.

When power is supplied to the gun 330 through the second power source supply 335 in operation S426, discharging is started, and thus plasma in which the argon gas and the oxygen gas are simultaneously ionized may be formed. Not all of oxygen molecules are ionized, and some amount of the oxygen molecules may be present in a molecular state while another amount of the oxygen molecules may be in an ionized state. According to one or more exemplary embodiments, plasma power may be provided within a range of 1 kw to 15 kw, and a temperature of a processing chamber may be changed based on the plasma power. In addition, a processing pressure may be within a range of 3 mTorr to 10 mTorr.

The ionized argon ions and the ionized oxygen ions are attracted to and accelerated toward the titanium target sample 344 which acts as a negative electrode by receiving a force of the electric field. The accelerated argon ions collide with the titanium target sample 344 and transfer energy to a surface of the target sample 344, and thus titanium atoms of the target sample 344 may be ejected.

The titanium atoms ejected from the target sample 344 have high energy and react with the oxygen gas injected inside the second vacuum chamber 310 as in the following Reaction Formula 4, and thus the photocatalyst thin film 170 having a titanium oxide component may be generated.

Reaction Formula 4 2Ti + O2 → 2TiO

The partially ionized oxygen ions attracted to and accelerated toward the titanium target sample 344 collide with the surface of the titanium target sample 344, receive electrons and are neutralized, and others react with titanium on the surface of the target sample 344 and titanium oxide is formed. Some of the titanium oxide generated by the reaction remains on the surface of the target sample 344 and also causes a change in the color of the target sample 344.

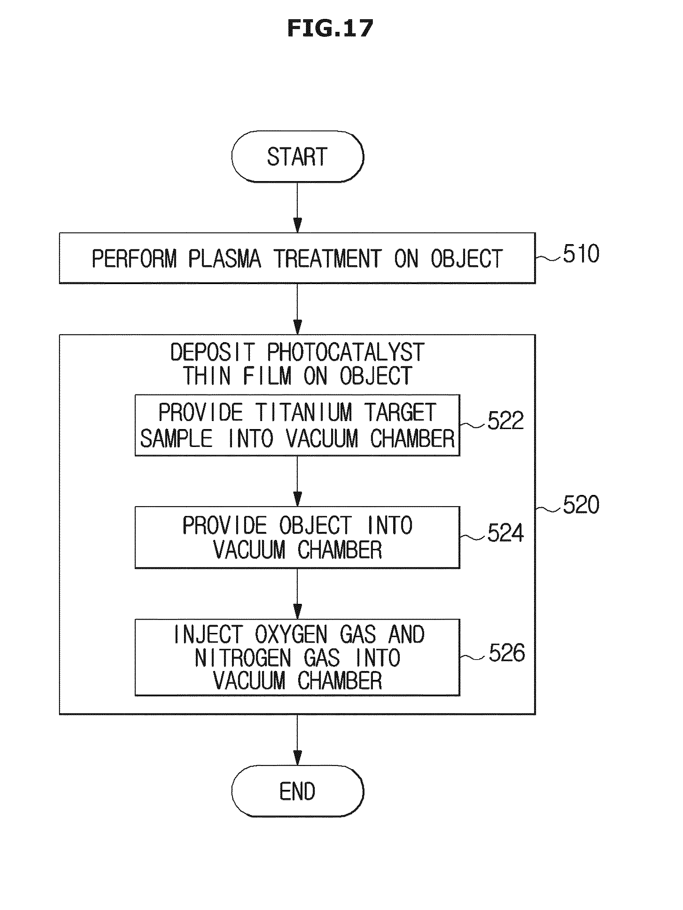

FIG. 17 is a flowchart for describing a method of manufacturing a display apparatus 100 according to another exemplary embodiment.

Referring to FIG. 17, the display apparatus 100 according to another exemplary embodiment may include performing a plasma treatment on an object (S510) and depositing a photocatalyst thin film 170 including nitrogen-doped titanium oxide on the object (S520). The depositing of the photocatalyst thin film 170, including the nitrogen-doped titanium oxide on the object (S520), may include providing a titanium target sample 344 into a vacuum chamber 310 (S522), providing the object into the vacuum chamber 310 (S524), and injecting an oxygen gas serving as a reaction gas inside the vacuum chamber 310 (S526). Hereinafter, a case in which a display panel 120 is provided as the object will be described as an example.

A difference between the present exemplary embodiment and that of FIG. 6 is that a nitrogen gas, which serves as a reaction gas in addition to the oxygen gas, is also injected. Hereinafter, repetitive descriptions of the plasma treatment process (S510), the providing of the titanium target sample 344 into the vacuum chamber 310 (S522), and the providing of the object inside the vacuum chamber 310 (S524), which are described above in FIG. 16 will be omitted.

In the present exemplary embodiment, once the display panel 120 is placed in the second vacuum chamber 310, the process in which the oxygen gas and a nitrogen gas are injected inside the second vacuum chamber 310 may be performed (S526). More specifically, control valve 326 is controlled while the second vacuum chamber 310 is maintained in a vacuum state by the second vacuum pump 314, and an argon gas, the oxygen gas, and the nitrogen gas may be injected into the second vacuum chamber 310.

In operation S526, when the titanium target sample 344 is used, the argon gas may be injected within a range of 60 sccm to 360 sccm and the oxygen gas may be injected within a range of 30 sccm to 180 sccm. In addition, the nitrogen gas for improving photocatalyst efficiency may be injected within a range of 30 sccm to 180 sccm. According to one or more exemplary embodiments, when a titanium oxide target sample is used, the argon gas and the nitrogen gas may be injected. More specifically, the argon gas may be injected within a range of 60 sccm to 360 sccm, and the nitrogen gas may be injected within a range of 30 sccm to 180 sccm.

When power is supplied to a gun 330 through a second power source supply 335, discharging is started, and thus plasma in which the oxygen gas and the nitrogen gas are simultaneously ionized may be formed. Not all oxygen molecules and nitrogen molecules are ionized, and some amount of the oxygen molecules and the nitrogen molecules may be present in a molecular state while another amount of the oxygen molecules and the nitrogen molecules may be in an ionized state. According to one or more exemplary embodiments, plasma power may be provided within a range of 1 kw to 15 kw, and a temperature of a processing chamber may be changed based on the plasma power. In addition a processing pressure may be provided within a range of 3 mTorr to 10 mTorr.

An argon ion, an oxygen ion, and a nitrogen ion, which are generated by the ionization are attracted to and accelerated toward the titanium target sample 344 which acts as a cathode by receiving a force of the electric field. The accelerated argon ions collide with the titanium target sample 344, transfer energy to a surface of the target sample, and thus, titanium atoms of the target sample 344 may be ejected.

The titanium atoms ejected from the target sample 344 have high energy and react with the oxygen gas and the nitrogen gas injected inside the second vacuum chamber 310, and thus the photocatalyst thin film 170 having a titanium oxide component and a titanium nitride component may be generated.

The partially ionized oxygen ions or the partially ionized nitrogen ions, which are attracted to and accelerated toward the titanium target sample 344 collide with the surface of the titanium target sample 344, receive electrons and are neutralized, and others react with titanium on the surface of the target sample 344 and titanium oxide or titanium nitride is formed. Some amount of the titanium oxide or some amount of the titanium nitride, which is generated by the reaction remains on the surface of the target sample 344 and causes a change of color of the target sample 344.

According to the above-described exemplary display apparatus and method of manufacturing the same, a display apparatus having a self-cleaning function and a sterilization function can be provided.

The display apparatus 100 and the method of manufacturing the same are described above. The scope of the inventive concept is not limited to the above-described embodiments, and is defined by the claims.

## Claims

1. A display apparatus comprising:
a housing comprising an exterior of the display apparatus;
a display panel coupled to the housing; and
a photocatalyst thin film formed on a surface of at least one of the housing and the display panel.

2. The display apparatus according to claim 1, wherein the photocatalyst thin film comprises a thin film of at least one of titanium oxide (TiO2) and nitrogen-doped titanium oxide (TiO2-xNx).

3. The display apparatus according to claim 1 or 2, wherein the display apparatus comprises one of a liquid crystal display apparatus, an organic light-emitting display apparatus, and a quantum dot display apparatus.

4. The display apparatus according to claim 1, 2 or 3, wherein the housing comprises at least one of a metal component and a plastic component.

5. The display apparatus according to claim 4, wherein the metal component is analuminum alloy component.

6. A display apparatus comprising:
a housing comprising an exterior of the display apparatus;
a display panel coupled to the housing; and
a photocatalyst thin film formed on a surface of the display panel.

7. The display apparatus according to claim 6, wherein the photocatalyst thin film comprises a thin film of at least one of titanium oxide (TiO2) and nitrogen-doped titanium oxide (TiO2-xNx) thin film.

8. The display apparatus according to claim 6, wherein the display apparatus comprises one of a liquid crystal display apparatus, an organic light-emitting display apparatus, and a quantum dot display apparatus.

9. The display apparatus according to claim 6, wherein the display panel comprises:
a liquid crystal display panel comprising:
a first substrate comprising field generating electrodes,
a second substrate comprising field generating electrodes, and
a liquid crystal layer interposed between the first substrate and the second substrate, wherein the photocatalyst thin film is formed on a surface of at least one of the first substrate and the second substrate.

10. The display apparatus according to claim 9, further comprising a backlight unit configured to direct light to the liquid crystal display panel,
wherein the surface on which the photocatalyst thin film is formed faces the backlight unit such that the light directed to the liquid crystal display panel is absorbed into the backlight unit.

11. A display apparatus comprising:
a housing comprising an exterior of the display apparatus;
a display panel coupled to the housing; and
a photocatalyst thin film formed on a surface of the housing.

12. The display apparatus according to claim 11, wherein the photocatalyst thin film comprises a film of at least one of titanium oxide (TiO2) and nitrogen-doped titanium oxide (TiO2-xNx).

13. The display apparatus according to claim 11, wherein the housing is comprises at least one of a metal component and a plastic component.

14. The display apparatus according to claim 13, wherein the metal component is an aluminum alloy component.
